# EUROPEAN PATENT SPECIFICATION

(11) **EP 2 601 528 B1**
(45) Date of publication and mention of the grant of the patent: **28.10.2015**
(21) Application number: 11738451.1
(22) Date of filing: 01.08.2011
(51) Int. Cl.: G01N 33/574, C12Q 1/68

(54) **NOVEL METHODS FOR PREDICTING THE RESPONSIVENESS OF A PATIENT AFFECTED WITH A TUMOR TO A TREATMENT WITH A TYROSINE KINASE INHIBITOR**
NEUE VERFAHREN ZUR VORHERSAGE DER REAKTIONSFÄHIGKEIT EINES TUMORPATIENTEN AUF EINE BEHANDLUNG MIT EINEM TYROSINKINASEHEMMER
NOUVEAUX PROCÉDÉS POUR LA PRÉVISION DE LA RÉACTIVITÉ D'UN PATIENT ATTEINT D'UNE TUMEUR À UN TRAITEMENT PAR UN INHIBITEUR DE LA TYROSINE KINASE

(30) Priority: 17.02.2011 EP 11305167; 02.08.2010 EP 10305852
(43) Date of publication of application: 12.06.2013
(73) Proprietor: INSERM (Institut National de la Santé et de la Recherche Médicale), 75013 Paris (FR); Icahn School of Medicine at Mount Sinai, New York, NY 10029-6574 (US)
(72) Inventor: GHINEA, Nicolae, 94000 Créteil (FR); RADU, Aurelian, New York, NY 10029 (US)
(74) Representative: Grünecker Patent- und Rechtsanwälte PartG mbB
(86) International application number: PCT/EP2011/063215
(87) International publication number: WO 2012/016948

(56) References cited:
- EP-A1- 2 090 322
- WO-A1-2010/007464
- US-A1- 2007 178 494
- US-A1- 2009 285 832
- CHEN GUANG ET AL: "Identification of p27/KIP1 expression level as a candidate biomarker of response to rapalogs therapy in human cancer.", JOURNAL OF MOLECULAR MEDICINE (BERLIN, GERMANY) SEP 2010 LNKD- PUBMED:20508912, vol. 88, no. 9, 28 May 2010 (2010-05-28), pages 941-952, XP002603689, ISSN: 1432-1440

## Description

### FIELD OF THE INVENTION:

The present invention relates to a method for predicting the responsiveness of a patient affected with a tumor to a treatment with a tyrosine kinase inhibitor (TKI), such as sunitinib.

### BACKGROUND OF THE INVENTION:

Angiogenesis is a well-known process involved in tumor growth and metastasis since such diseases are known to be associated with deregulated angiogenesis. Angiogenesis is a privileged target of several agents in the treatment of solid and hematologic malignancies. Among new concepts developed to improve the management of tumors, molecules targeting the VEGF signaling have been developed, and namely molecules inhibiting VEGF receptors (VEGF-R), which belong to the class of tyrosine kinase inhibitors (TKI). VEGF-R is indeed a receptor tyrosine kinase (RTK) that has been shown to be not only a key regulator of normal cellular processes but also to have a critical role in the development and progression of many types of cancer. There exist two different tyrosine kinase VEGF-Rs at the cell surface, VEGF-R1 (Flt-1) and VEGF-R2 (KDR/Flk-1) possessing a single transmembrane spanning region and an intracellular portion containing a tyrosine-kinase domain. Tyrosine kinases are particularly important today because of their implication in the treatment of cancer. Indeed, phosphorylation of proteins by kinases is an important mechanism in signal transduction for regulation of enzyme activity.

Therefore most cancer patients fail to respond to a TKI treatment and yet, no predictive factor or biomarker of the response to TKI has been identified. Moreover, there are no clinical methods capable of discriminating and/or predicting a preferential efficacy of TKI VEGFR2 inhibitors (sunitinib, sorafenib) compared with bevacizumab (anti-VEGF monoclonal antibody), or with mTOR inhibitor (temsirolimus).

For example, sunitinib has proven efficacy in metastatic renal cell carcinoma (mRCC) but the molecular mechanisms underlying the clinical response to this drug remain unclear. Yet, renal cell carcinoma (RCC) represents 3% of malignancies with 88,400 in 2008 in Europe and, 57,760 new cases diagnosed in 2009 in the United States and during last decades, this incidence has constantly increased. At the time of diagnosis, about 30% of RCC are metastatic. For a long time, mRCC has been considered as resistant to the majority of treatments. The management was based on nephrectomy, and the use of immunotherapy (interleukin-2, interferon-α), often poorly tolerated and efficient. Thus, before the introduction of antiangiogenic therapies, the median survival time of mRCC was about twelve months. In first-line therapy, sunitinib significantly improved progression-free survival (PFS) by reducing the risk of relapse of 58% compared to interferon-α. However, no predictive clinical or biological factors of response have been identified allowing a better selection of RCC patients for sunitinib therapy.

Therefore, if VEGF-R TKI such as sunitinib provide considerable promise for patients, there is a crucial need for a better selection of patients. Indeed, tumors with close characteristics can present opposite behavior with either important and long regressions, or very short-term progressions. Contrary to breast cancer where a target such as HER2 has been discovered prior to anti-HER2 therapies, VEGF-R TKI may also inhibit other tyrosine kinases, which impact other cascades of signaling pathways. Thereby, the optimization of their efficiency is based on a correlation between response to treatment and individual tumor signatures. Indeed, despite recent progress in antitumoral drug development, the treatment of patients affected with a tumor remains a challenge both in term of clinical- and cost-effectiveness.

Furthermore only about one third of the RCC patients respond to the Sutent treatment [Motzer et al., 2007]. Sutent has significant adverse side effects, which are experienced by the patients for a relatively long period (a typical treatment session takes 3 months). It would be useful if only patients that would respond to Sutent would be subjected to the treatment. Unfortunately no criteria are currently available to predict, before a full course of treatment is applied, if a patient would benefit of it or not. As a consequence two thirds of the patients experience the side effects with no benefit from the treatment.

Thus, considering the number of side effects and costs of treatment, prediction of early antitumoral drug response during therapy or even before starting therapy is needed. However, there are no well-established biomarkers, which may improve the selection of patients who may benefit from the treatment.

The purpose of the present invention is therefore to address this need by providing a new reliable method for predicting whether a patient affected with a tumor is responder or no responder to a treatment with a TKI.

### SUMMARY OF THE INVENTION:

The invention relates to a method for predicting the responsiveness of a patient affected with a tumor to a treatment with a tyrosine kinase inhibitor (TKI), comprising a step of measuring the expression level of FSHR in a biological sample from said patient, wherein said sample is - selected from the group consisting of a resected tumor, or a biopsy, and more specifically from tissue tumor sample and a step of comparing the expression level of FSHR with control reference values obtained from responder and non-responder group of patients.

The invention also relates to a TKI for use in the treatment of a patient affected with a tumor, comprising the diagnostic step of predicting the responsiveness of the patient to treatment with a TKI according to the method of the invention.

The invention also relates to the use of FSHR as a surrogate marker for predicting the responsiveness of a patient affected with a tumor to a treatment with a TKI.

### DETAILED DESCRIPTION OF THE INVENTION:

The inventors have shown that the responsiveness of patients affected with a tumor receiving a treatment with a tyrosine kinase inhibitor (TKI) be accurately predicted by determining the level of expression of a marker consisting of follicle stimulating hormone receptor (FSHR).

### Definitions:

As used herein, the term "FSHR" has its general meaning in the art and denotes the gene encoding the FSH (follicle stimulating hormone) receptor to which the methods of the invention apply. The FSHR protein has 678 amino acids after signal sequence cleavage. Since the FSHR was found to be expressed by endothelial cells of microvessels associated with tumors, the receptor is also designated Vascular endothelial FSHR or VE-FSHR. The sequence of the human FSHR gene is available in the art namely in the database under the accession number GeneID: 2492.

A cDNA encoding the human FSH receptor (FSHR) has been isolated and sequenced by Minegish et al., 1990. FSHR is a transmembrane receptor that interacts with the follicle stimulating hormone (FSH) and represents a G protein-coupled receptor (GPCR). FSH exerts its biological role by binding to the plasma membrane FSH receptor (FSHR).

The amino-acid sequence of the FSHR is available in the SWTSSPROT database under the accession number P23945.

The term "expression" when used in the context of the expression of a gene or a nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by various processing such as capping, polyadenylation, methylation, and editing, and proteins modified by, for example, methylation, acetylation, phosphorylation, ubiquitination, SUMOylation, ADP-ribosylation, myristilation, and glycosylation.

The term "surrogate marker" is, in the sense of the invention, a marker which is differentially expressed in responder patients to treatment with a TKI, such as sunitinib comparatively to non responder patients to the same treatment. Specifically, a surrogate marker may be any gene expression product which is differentially expressed in responder patients when compared to non responder patients. A surrogate marker can be a polynucleotide, a protein, a peptide, or any gene expression product, but is preferably a mRNA or a protein expression product. The surrogate markers described herein is namely useful for predicting the responsiveness of a patient affected with a tumor to a treatment with a TKI. Thus, the term "surrogate marker" as used herein refers to those biological molecules which are differentially expressed in response to a treatment with a TKI.

According to the invention, the term "patient", is intended for a human or non-human mammal affected or likely to be affected with a tumor.

The term "responder" patient, or group of patients, refers to a patient, or group of patients, who show a clinically significant relief in the disease when treated with a TKI. Conversely, a "non responder patient" or group of patients, refers to a patient or group of patients, who do not show a clinically significant relief in the disease when treated with a TKI. When the disease is metastatic renal cell carcinoma (mRCC), a preferred responder group of patients that provides for the control reference values is a group that shows at least 30% decrease in the sum of the longest diameter of such target lesions (partial response) according to RECIST criteria [Therasse P et al 2000 Eisenhauer EA et al 2009], three months after 2 cycles (four weeks ON and two weeks OFF) of treatment with a TKI, preferably sunitinib, and preferably a group of patients showing the disappearance of all target lesions (complete response).

After being tested for responsiveness to a treatment with a TKI, the patients may thus be prescribed with said TKI, with reasonable expectations of success.

The terms "tyrosine kinase inhibitor" or " TKI" as used herein refer to any compound, natural or synthetic, which results in a decreased phosphorylation of the tyrosine present on the intracellular domain of receptor tyrosine kinases (RTK) such as growth factor receptors. Accordingly, TKI may be a multi-target tyrosine kinase inhibitor and may thus inhibit the epidermal growth factor (EGF) receptor family (such as HER-2); the insulin-like growth factor (IGF) receptor family (such as IGF-1 receptor); the platelet-derived growth factor (PDGF) receptor family, the colony stimulating factor (CSF) receptor family (such as CSF-1 receptor); the C-Kit receptor and vascular endothelial growth factor (VEGF) receptor family (such as VEGF-R1 (Flt-1) and VEGF-R2 (KDR/Flk-1)).

Preferably, the tyrosine kinase inhibitor is a VEGF-R tyrosine kinase. Typically, the efficacy of the compounds of the invention as inhibitors of VEGF-R tyrosine kinase activity can be demonstrated as described in US patent application 2003/0064992.

FSH-FSHR signaling can induce or increase EGFR1 (HER1 / ErbB1) and EGFR2 (HER2 / ErbB2) expression or signaling in certain cell types [Zhang et al., 2009]. EGFR1 is upregulated in tumor ECs and targeting of EGFR1 activity in blood vessels inhibits tumor growth in a cancer animal model. Patients whose tumor cells do not significantly express EGFR1 respond to EGFR1 inhibitors, which can be explained by the effect of the inhibitors on the EGFR expressed by the tumor ECs. EGFR2 is also known to be expressed by some EC types. Based on these observations it is expected that, as in the case of Sutent, FSHR expression by the tumor ECs would predict the response to treatment with EGFR1 inhibitors (e.g. gefitinib (Iressa) and erlotininb (Tarceva)) and inhibitors of EGFR2 (e.g. lapatininb, which also inhibits EGFR1).

FSH-FSHR signaling activates mammalian target of rapamycin (mTOR) signaling in granulosa cells [Kayampilly and Menon, 2007]. In cancer, mTOR is frequently hyperactivated and is a clinically validated target for drug development. mTOR signaling occurs in ECs. mTOR inhibitors have potent antiangiogenic properties in ECs. In this case too it is expected that, as for Sutent, FSHR expression by the tumor ECs could predict the response to treatment with mTOR inhibitors (e.g. everolimus, sirolimus and tensirolimus).

FSHR is also expressed in some tumor types by the tumor cells (kidney, breast and pancreas). It is expected that the level of FSHR expression by the tumor cells in such tumors could be used to predict the response to inhibitors of VEGFR and of other kinases inhibited by Sutent, and to inhibitors of EGFR1, EGFR2 and mTOR.

### Methods for predicting the responsiveness of a patient according to the invention:

A first aspect of the invention consists of a method for predicting the responsiveness of a patient affected with a tumor to a treatment with a tyrosine kinase inhibitor (TKI), comprising a step of measuring the expression level of FSHR in a biological sample from said patient, wherein said sample is selected from the group consisting of a resected tumor, or a biopsy, and more specifically from tissue tumor sample and a step of comparing the expression level of FSHR with control reference values obtained from responder and non-responder group of patients. In a particular embodiment, methods of the invention are suitable for predicting the responsiveness of a patient affected with a tumor to a pharmaceutical treatment with a TKI, wherein said TKI is selected in the group consisting of axitinib, cediranib, imatinib, dasatinib, nilotinib, pazopanib, semaxanib, sorafenib, sunitinib, vandetanib, vatalanib), and mTOR inhibitors (e.g. everolimus, sirolimus, tensirolimus).

In preferred embodiments, the TKI is sunitinib or N-[2-(diethylamino)ethyl]-5-[(Z)-(5-tluoro-1,2-dihydro-2-oxo-3H-indol-3-ylidine)methyl]-2,4-dimethyl- 1H-pyrrole-3 carboxamide preferably the malic acid addition salt thereof (marketed as SUTENT® by Pfizer and previously known as SU11248).

Patients who have been clinically diagnosed as being affected with any tumor sensitive to a TKI inhibitor are of particular interest in the invention. However in a preferred embodiment the patient is affected with a solid tumor. According to this embodiment, the solid tumor is selected in the group consisting of kidney cancer such as renal cell carcinoma (RCC) and metastatic renal cell carcinoma (mRCC), stomach cancer and gastrointestinal cancer such as gastrointestinal stromal tumor (GIST), hepatic cancer such as hepatocellular carcinoma (HCC), lung cancer, colorectal cancer, breast cancer, head and neck cancer, melanoma, prostate cancer and pancreatic cancer such as pancreatic neuroendocrine tumor and any solid tumor in which sutent showed efficacy.

In one preferred embodiment, the solid tumor is a renal cell carcinoma (RCC) or a metastatic renal cell carcinoma (mRCC).

As demonstrated in the following examples and in WO2009103741 for respectively renal cell carcinoma and for prostate cancer, FSHR is expressed in blood vessels in tumors, more particularly malignant tumors, but not in normal tissue

In another embodiment, the patient is affected with a haematological malignancy. According to this embodiment, the haematological malignancy is selected in the group consisting of multiple myeloma, non-Hodgkin's lymphoma, acute and chronic leukemia (e.g. acute myeloid leukemia (AML).

Samples that may be used for performing the methods according to the invention encompass surgically removed tumors and biological sample obtained from a patient, including any fluids, tissues, cell samples, organs, biopsies, etc.

The biological sample results from a resected tumor or a biopsy, and more specifically from a tumor tissue sample that comprises blood vessels..

Control reference values are easily determinable by the one skilled in the art, by using the same techniques as for determining the level of FSHR in biological samples previously collected from the patient under testing.

As indicated above, a first control reference value consists of the maximal expression level value of the relevant marker that has been determined in a group of patients affected with a tumor that are stable or not responsive to a treatment with a TKI.

A second control reference value consists of the minimal expression value of the relevant marker genes that has been determined in a group of patients affected with a tumor that are responsive to a treatment with a TKI.

At the end of the method according to the invention, an expression value found for the marker genes in the patient tested inferior to the first control reference value above indicates that the patient tested consists of a stable or non-responder to the treatment with a SKI.

At the end of the method according to the invention, an expression value found for the marker genes in the patient tested superior to the second control reference value above indicates that the patient tested consists of a responder patient to the treatment with a TKI.

In a particular embodiment, the method of the invention is performed by determining the ratio between the density of the vessels that show a FSHR signal and the total number of vessels in the tumor (for instance the vessels positive for von Willebrand factor (vWF)). The patients will be categorized as "responsive" to the treatment with a TKI if the ratio is above 0.30, and "non-responsive" or "stable" to the treatment with a TKI if the ratio is below 0.225.

Accordingly, the present invention relates to a method for predicting the responsiveness of a patient affected with a tumor to a treatment with a tyrosine kinase inhibitor comprising the step consisting of determining the density of FSHR-positive vessels in the tumor tissue sample obtained from the patient.

As used herein the term "FSHR-positive vessels" means blood vessels for which endothelial cells express FSHR

Density of FSHR-positive vesselmay be determined according to any well known method in the art. Typically the density is performed in an IHC assay under microcospy as described in the Example.

Total RNAs or proteins can be easily extracted therefrom. The sample may be treated prior to its use, e.g. in order to render nucleic acids or proteins available. Techniques of cell or protein lysis, concentration or dilution of nucleic acids, are known by the skilled person.

### Determination of the expression level of the marker genes by quantifying mRNAs

Determination of the expression level of a gene such as FSHR can be performed by a variety of techniques. Generally, the level as determined is a relative expression level.

More preferably, the determination comprises contacting the sample with selective reagents such as probes, primers or ligands, and thereby detecting the presence, or measuring the amount, of polypeptides or nucleic acids of interest originally in the sample. Contacting may be performed in any suitable device, such as a plate, microtiter dish, test tube, well, glass, column, etc. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc.

In a particular embodiment, the expression level may be determined by determining the amount of mRNA. Such method may be suitable to determine the expression levels of FSHR genes in a nucleic sample such as a tumor biopsy.

Methods for determining the amount of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then determined by hybridization (e. g., Northern blot analysis).

Alternatively, the extracted mRNA may be subjected to coupled reverse transcription and amplification, such as reverse transcription and amplification by polymerase chain reaction (RT-PCR), using specific oligonucleotide primers that enable amplification of a region in the FSHR genes. Preferably quantitative or semi-quantitative RT-PCR is preferred. Real-time quantitative or semi-quantitative RT-PCR is particularly advantageous. Extracted mRNA may be reverse-transcribed and amplified, after which amplified sequences may be detected by hybridization with a suitable probe or by direct sequencing, or any other appropriate method known in the art.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein are useful as hybridization probes or amplification primers. It is understood that such nucleic acids need not to be identical, but are typically at least about 80% identical to the homologous region of comparable size, more preferably 85% identical and even more preferably 90-95% identical.

In certain embodiments, it is advantageous to use nucleic acids in combination with appropriate means, such as a detectable label, for detecting hybridization. A wide variety of appropriate labels are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

In a particular embodiment, the method of the invention comprises the steps of providing total RNAs obtained from the biological sample of the patient, and subjecting the RNAs to amplification and hybridization to specific probes, more particularly by means of a quantitative or semi-quantitative RT-PCR.

When quantifying FSHR transcripts by RT-PCR, according to the invention, the result is preferably expressed as a relative expression of said isoform, having regard to at least one gene with a constant expression level, for example a housekeeping gene such as PPIA (peptidylprolyl isomerase A), TBP (TATA-box binding protein), GADPDH (Glyceraldehyde-3-phosphatedehydrogenase), actine, GUS (beta-glucuronidase), RPLP0 (Ribosomal protein, large, P0) and TFRC (transferrin receptor).

Total RNAs can be easily extracted from a biological sample. For instance, the biological sample may be treated prior to its use, e.g. in order to render nucleic acids available. Techniques of cell or protein lysis, concentration or dilution of nucleic acids, are known by the skilled person.

In another embodiment, the expression level may be determined by DNA microarray analysis. Such DNA microarray or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art

In this context, the invention further provides a DNA microarray comprising a solid support onto which nucleic acids that are specific for FSHR gene nucleic acid expression products (i.e. mRNA or cDNA) are immobilized.

### Determination of the expression level of the marker genes by quantifying proteins

Other methods for determining the expression level of the said marker gene, namely FSHR, include the determination of the quantity of polypeptides encoded by said genes that is found in the sample previously collected from the patient to be tested. In particular, such methods may be particularly suitable to determine the quantity of FSHR proteins in a sample like a tumor biopsy.

Such methods comprise contacting a sample with a binding partner capable of selectively interacting with a marker protein present in the sample. The binding partner is generally an antibody that may be polyclonal or monoclonal, preferably monoclonal.

Monoclonal antibodies for FSHR, are described, for example, in Vannier et al., 1996.

The presence of the protein may be detected by using standard electrophoretic and immunodiagnostic techniques, including immunoassays such as competition, direct reaction such as immunohistochemistry, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads or magnetically responsive beads.

More particularly, an ELISA method may be used, wherein the wells of a microtiter plate are coated with a set of antibodies against the proteins to be tested. A biological sample containing or suspected of containing the marker protein is then added to the coated wells. After a period of incubation sufficient to allow the formation of antibody-antigen complexes, the plate (s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

Alternatively an immunohistochemistry (IHC) method may be preferred. IHC specifically provides a method of detecting targets in a sample or tissue specimen in situ. The overall cellular integrity of the sample is maintained in IHC, thus allowing detection of both the presence and location of the targets of interest. Typically a sample is fixed with formalin, embedded in paraffin and cut into sections for staining and subsequent inspection by light microscopy. Current methods of IHC use either direct labeling or secondary antibody-based or hapten-based labeling. Examples of known IHC systems include, for example, EnVision(TM) (DakoCytomation), Powervision(R) (Immunovision, Springdale, AZ), the NBA(TM) kit (Zymed Laboratories Inc., South San Francisco, CA), HistoFine(R) (Nichirei Corp, Tokyo, Japan).

In particular embodiment, a tissue section may be mounted on a slide or other support after incubation with antibodies directed against the proteins encoded by the genes of interest. Then, microscopic inspections in the sample mounted on a suitable solid support may be performed. For the production of photomicrographs, sections comprising samples may be mounted on a glass slide or other planar support, to highlight by selective staining the presence of the proteins of interest.

In some embodiments, an IHC staining procedure may comprise steps such as: cutting and trimming tissue, fixation, dehydration, paraffin infiltration, cutting in thin sections, mounting onto glass slides, baking, deparaffination, rehydration, antigen retrieval, blocking steps, applying primary antibodies, washing, applying secondary antibodies (optionally coupled to a suitable detectable label), washing, counter staining, and microscopic examination.

### Use of surrogate marker for predicting the responsiveness of a patient:

Another aspect of the invention is the use of FSHR as a surrogate marker for predicting the responsiveness of a patient affected with a tumor to a treatment with a TKI.

According to this aspect, this use of FSHR as a surrogate marker comprise a step of measuring the expression level of the FSHR gene in a biological sample from said patient and a step of comparing the expression level of FSHR with control reference values obtained from responder and non-responder group of patients.

In this embodiment, control reference values are determined as for the method of prediction of the invention.

In an other embodiment, the expression level of FSHR is performed by determining the ratio between the density of the vessels that show a FSHR signal and the total number of vessels in the tumor (for instance the vessels positive for von Willebrand factor (vWF)).

A ratio above 0.30 of the number of vessels that are FSHR-positive per total number of vessels in the tumor is indicative for said patient to be a responder to the treatment with a TKI, and a ratio below 0.225 is indicative for said patient to be stable or non-responsive to the treatment with a TKI.

Accordingly, the present invention relates to a method for predicting the responsiveness of a patient affected with a tumor to a treatment with a tyrosine kinase inhibitor comprising the steps consisting of i) determining the density of FSHR-positive vessels in the tumor tissue sample obtained from the patient and ii) comparing the density at step i) with a reference value wherein a difference between density and said reference value is indicative whether the patient will responds to the treatment with said tyrosine kinase inhibitor.

The expression level of FSHR may be determined by quantifying mRNAs or by quantifying proteins according to the methods as described above.

In a particular embodiment, the use of FSHR as surrogate biomarkers for predicting the responsiveness of a patient affected with a metastatic renal cell carcinoma (mRCC) to a treatment with a TKI.

In another particular embodiment, the use of FSHR as surrogate biomarker for predicting the responsiveness of a patient affected with a tumor to a treatment with sunitinib.

In a preferred embodiment, the use of FSHR as surrogate biomarker for predicting the responsiveness of a patient affected with a metastatic renal cell carcinoma to a treatment with sunitinib.

### TKI for use in Therapeutic methods:

A further aspect of the invention relates to TKI for use in a method for the treatment of a tumor in a patient, such as a solid tumor or a hematologic malignancy (see claim 9).

In the context of the invention, the term "treating" or "treatment", as used herein, means reversing, alleviating, inhibiting the progress of, or preventing the disorder or condition to which such term applies, or one or more symptoms of such disorder or condition.

Said method comprises the following step:
a) determining whether a patient affected with a tumor is a responder or a non responder to a treatment with a TKI, by performing the method as above described.
b) The TKI is then to be administered to said patient, if said patient has been determined as consisting of a responder, at step a) above.

In one preferred embodiment, the TKI is sunitinib.

In another preferred embodiment, the tumor affecting the patient is as renal cell carcinoma (RCC) or a metastatic renal cell carcinoma (mRCC).

The invention will be further illustrated by the following figures and examples. However, these examples and figures should not be interpreted in any way as limiting the scope of the present invention.

### FIGURES:

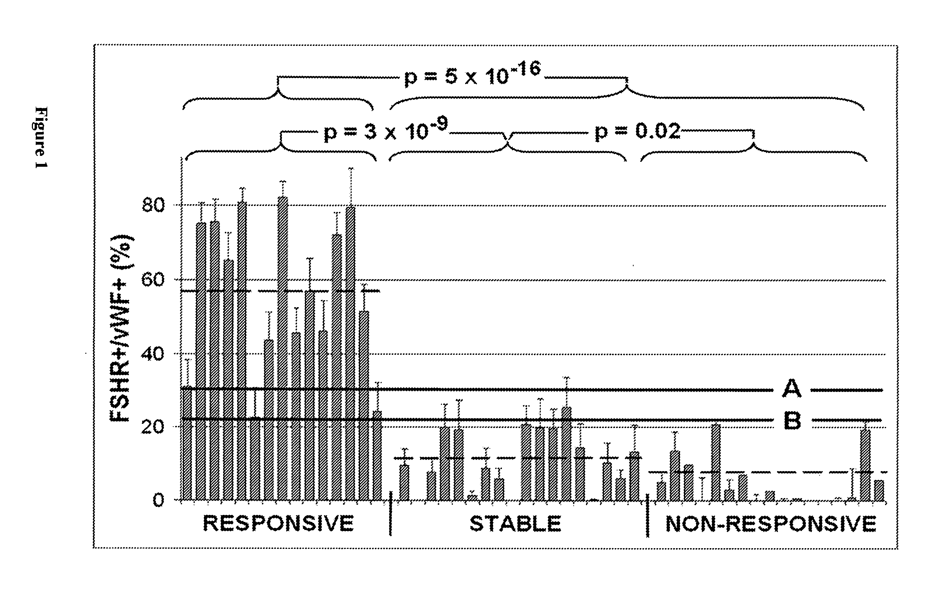

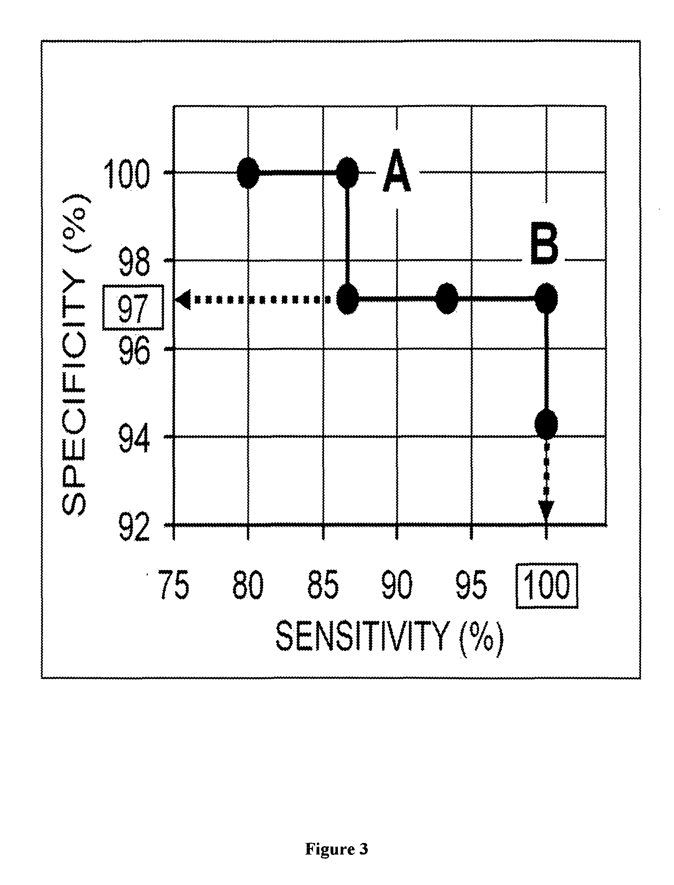

**Figure 1****.** The ratio between the density of the vessels that show a FSHR signal and vessels positive for von Willebrand factor (vWF) is correlated with the response of the patients to subsequent treatment with Sutent. The bars correspond to 15 patients who responded to the treatment, 18 patients who were stable and 17 patients who did not respond. Errors bars: standard errors of the means computed for 20 microscopic fields for each patient. The dashed lines correspond to the averages for the 3 groups of patients (56.8+/- 5.4%, 11.4 +/-2% and 7.3 +/- 0.7% for the responsive, stable and non- responsive? patients, respectively. The solid lines marked A and B correspond to the 2 thresholds (30% and 22.5%, respectively) used in the points A and B in Fig. 3 to determine the sensitivities and the specificities of discriminating between the patients who are responsive vs. the combined stable or non-responsive set. (As shown in Fig. 3 for threshold A the sensitivity is 87% and the specificity is 100%, while for threshold B the sensitivity is 100% and the specificity is 97%).

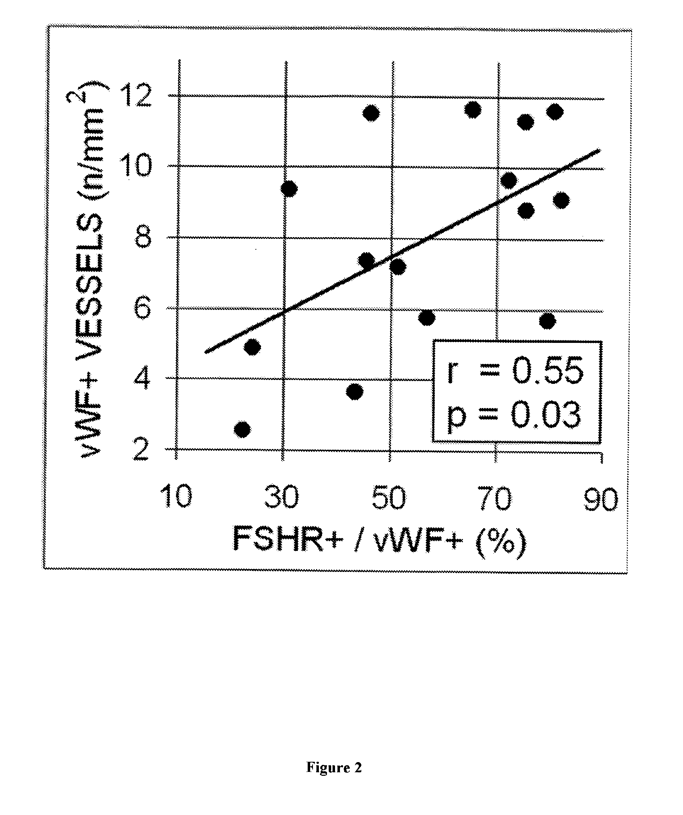

**Figure 2****.** A significant positive correlation (r=0.55, p = 0.03) for the responsive patients exists between the percentage of FSHR+ vessels and the total density of vessels (vWF+ /mm²).
**Figure 3**. The ratio of the density of FSHR-stained vessels divided by the density of vWF-stained vessels predicts with high sensitivity and selectivity the patients who will be responsive to Sutent. Horizontal axis: sensitivity (%); vertical axis: specificity (%). For point A (for a threshold of the FSHR+ / vWF+ stained vessels = 30%), the sensitivity is 87% and the specificity is 100% (i.e. 87% of the patients who will respond are correctly predicted, and none of the patients who will be stable or non-responsive are incorrectly predicted to be responsive); for point B (FSHR+/vWF+ = 22.5%) the sensitivity is 100% and the specificity is 97% (dashed arrows) (i.e. all patients who will respond are correctly predicted, and only 3% of the stable or non-responsive patients are incorrectly predicted to be responsive.

### EXAMPLE: FSHR LEVEL EXPRESSION IN A RENAL TUMOR BIOPSY OF PATIENTS AFFECTED BY A METASTATIC RENAL CELL CARCINOMA PREDICTS RESPONSIVENESS TO SUNITINIB.

### Material & Methods

All patients were diagnosed with advanced metastatic renal cell clear cell carcinoma and subjected to surgery for removal of the primary tumor. The tumor tissue was fixed in formaldehyde, and embedded in paraffin. Sections were cut and stained for the FSH receptor using the monoclonal antibody FSHR323, followed by a secondary HRP- or Alexa555-coupled antibody. The density of vessels in the tumor tissue was determined by microscopy using a 20 x objective. Starting at the border between the normal and the tumor tissue and moving towards the interior of the tumor, digital photographs were taken of every other field. The number of FSHR-positive vessels in each image was counted in 20 images from each tumor.

The patients were subjected to Sutent treatment for >= 3 months with a dose of 50 mg /day for 4 weeks followed by 2 weeks off. Depending on the effects of the treatment, at the end of the 3 months therapy course the patients were designated as "responsive", "stable" or "non-responsive" according to the criteria described in [Eisenhauer et al., 2009]. The patients were categorized as "responsive" if there was at least a 30% decrease in the sum of the diameters of the lesions, and "non-responsive" if the sum of the lesion sizes increased by at least 20%. Patients who did not meet the criteria for any the two categories were categorized as "stable".

### Results

50 patients treated with Sutent were analyzed: 15 who were "responsive" to the Sutent treatment, 18 who were "stable" and 17 who were "non-responsive".

No significant differences in the total density of vessels per mm² among the 3 groups were found: the densities of vWF-positive vessels are: 49.1+/- 4.9, 42.7 +/- 4.4 and 46.7 +/-5.4 (average +/- SEM) for the responsive, stable and non-responsive patients, respectively (p = 0.16 for responsive vs. stable and p = 0.68 for stable vs. non-responsive).

Substantially more FSHR-positive vessels were found for the patients who responded to the treatment (data not shown).

Subsequent measurement revealed that the 3 groups of patients were better differentiated by the ratio between the FSHR-positive vessel density and the total vessel density, detected as vWF-positive vessels, than by the density of FSHR-positive vessels alone. Ratios were measured on double immunofluorescence images.

The ratios for the 3 groups of patients are shown in Fig. 1. In the group of patients that responded to the treatment, the proportion of FSHR-stained vessels was on average 5 fold higher than in the stable group (56.8%+/-5.4 (SEM) vs. 11.4%+/-2.0, respectively), and almost 8 fold higher than in the non-responsive group (7.3% +/- 0.7) (p = 3 x 10⁻⁹ for the difference between responsive and stable patients, and p = 0.5 x 10⁻¹⁶ for the difference between responsive and non-responsive patients (t-test, 2 tails, equal variance). The difference between the stable and non-responsive groups was significant at p = 0.02.

A significant positive correlation (r = 0.55, p = 0.03) exists for the responsive donors between the percentage of FSHR-positive vessels and the total density of vessels (Fig. 2). The correlation does not occur for the stable or for the non-responsive patients (r = 0.11, p = 0.66 and r = 0.33, p = 0.19, respectively). A potential explanation is that FSHR contributes to tumor angiogenesis, so that the higher the percentage of FSHR stained vessels the higher the vessel density in the tumors. It is not clear however why this correlation is detectable only within the set of patients that respond to Sutent. An alternative explanation for the correlation is that FSHR expression is induced by a diffusible factor secreted by the tumor blood vessels, so that a higher density of vessels leads to a higher regional concentration of the FSHR-inducing factor. This hypothesis does however not explain why the correlation is valid only within the responsive set of patients.

The correlation between the density of FSHR-stained vessels and the response to Sutent provides the possibility of predicting the outcome of treatment with Sutent based on the density of FSHR vessels in the primary tumor. The potential of such method can be assessed based on the graph shown in Fig. 3.

FSHR expression was also visible by immunohistochemistry in the tumor cells in approx. 50% of the tumors analyzed (data not shown). Tumors in which FSHR is expressed by the tumor cells are equally present in all three sets of patients - there is no correlation between the response to Sutent and the presence of FSHR in the tumor cells. This observation is in agreement with the conclusion of a recent study that Sutent acts primarily on tumor endothelium rather than tumor cells to inhibit the growth of renal cell carcinoma.

The excellent correlation between FSHR expression and response to Sutent could be explained if FSH/FSHR was the only major system that activates VEGFR2, which in turn would imply that the normal VEGFR2 activator, VEGF, has a minor contribution. Clinical trials support this possibility. Bevazucimab, a monoclonal humanized antibody against circulating VEGF was evaluated in several studies in patients with RCC. BEV alone showed activity only as second-line treatment and only in cytokine-refractory patients [Yang et al., 2003]. The AVOREN trial showed a median Overall Survival (OS) of 23.3 months with BEV + INFalpha and 21.3 months for INFalpha plus placebo with no statistically significant difference [Escudier et al., 2007]. Nor did the survival difference reach statistical significance in the CALGB 90206 [Rini et al., 2008] trial where the OS was 18.3 months favoring BEV + INFalpha vs. 17.4 months for INFalpha + placebo. These studies indicate that VEGF has a marginal involvement in RCC progression.

### REFERENCES:

Throughout this application, various references describe the state of the art to which this invention pertains.
Eisenhauer E. A. , P. Therasse, J. Bogaerts, L.H. Schwartz, D. Sargent, R. Ford, J. Dancey, S. Arbuck, S. Gwyther, M. Mooney, L. Rubinstein, L. Shankar, L. Dodd, R. Kaplan, D. Lacombe, J. Verweij. 2009. New response evaluation criteria in solid tumours: Revised RECIST guideline (version 1.1). Eur J Cancer 45: 228-247.
Kayampilly PP. Menon KM. 2007. Follicle-stimulating hormone increases tuberin phosphorylation and mammalian target of rapamycin signaling through an extracellular signal-regulated kinase-dependent pathway in rat granulosa cells. Endocrinology. 148:3950-7.
Motzer RJ, Hutson TE, Tomczak P, Michaelson MD, Bukowski RM, Rixe O, Oudard S, Negrier S, Szczylik C, Kim ST, Chen I, Bycott PW, Baum CM, Figlin RA. 2007. Sunitinib versus interferon alfa in metastatic renal-cell carcinoma. N Engl J Med 356 :115-124.
Therasse P, Arbuck SG, Eisenhauer EA. 2000. New guidelines to evaluate the response to treatment in solid tumors. J Natl Cancer Inst 92:205-16.
Vannier B, Loosfelt H, Meduri G, Pichon C, Milgrom E. 1996. Anti-human FSH receptor monoclonal antibodies: immunochemical and immunocytochemical characterization of the receptor. Biochemistry 35: 1358-66.
Zhang Z, Jia L, Feng Y, Zheng W. 2009. Overexpression of follicle-stimulating hormone receptor facilitates the development of ovarian epithelial cancer. Cancer Lett 278:56-64.

## Claims

1. A method for predicting the responsiveness of a patient affected with a tumor to a treatment with a tyrosine kinase inhibitor (TKI), comprising a step of measuring the expression level of FSHR in a biological sample from said patient, wherein said sample is selected from the group consisting of a resected tumor, or a biopsy, and more specifically from tissue tumor sample and a step of comparing the expression level of FSHR with control reference values obtained from responder and non-responder group of patients.

2. The method according to claim 1 , wherein the TKI is selected in the group consisting of axitinib, cediranib, dasatinib, imatininb, nilotinib, pazopanib, semaxanib, sorafenib, sunitinib, vandetanib, vatalanib, everolimus, sirolimus and tensirolimus.

3. The method according to claim 2, wherein the TKI is sunitinib.

4. The method according to anyone claims 1 to 3, wherein the tumor is a solid tumor.

5. The method according to claim 4, wherein the solid tumor is selected in the group consisting of kidney cancer such as renal cell carcinoma (RCC), stomach cancer and gastrointestinal cancer such as gastrointestinal stromal tumor (GIST), hepatic cancer such as hepatocellular carcinoma (HCC), breast cancer, lung cancer, colorectal cancer, melanoma, prostate cancer and pancreatic cancer such as pancreatic neuroendocrine tumor.

6. The method according to claim 5, wherein the renal cancer RCC is a metastatic RCC.

7. The method of any of claims 1 to 6, wherein the level of FSHR is determined by quantifying the level of the proteins encoded in the sample.

8. The method of any of claims 1 to 6 wherein the level of FSHR is determined by quantifying the level of mRNA in the sample.

9. A TKI for use in the treatment of a patient affected with a tumor, comprising the diagnostic step of predicting the responsiveness of the patient to treatment with a TKI according to claims 1-8.

10. Use of FSHR as surrogate markers for predicting the responsiveness of a patient affected with a tumor to a treatment with a TKI.

11. A method for predicting the responsiveness of a patient affected with a tumor to a treatment with a tyrosine kinase inhibitor comprising the step consisting of determining the density of FSHR-positive vessels in the tumor tissue sample obtained from the patient.

12. The method according to claim 11 which comprises the steps consisting of i) determining the density of FSHR-positive vessels in the tumor tissue sample obtained from the patient and ii) comparing the density at step i) with a reference value wherein a difference between density and said reference value is indicative whether the patient will responds to the treatment with said tyrosine kinase inhibitor.

## Patentansprüche

1. Ein Verfahren zur Vorhersage der Reaktionsfähigkeit eines mit einem Tumor belasteten Patienten auf eine Behandlung mit einem Tyrosinkinaseinhibitor (TKI), umfassend
einen Schritt des Messens des Expressionsniveaus von FSHR in einer biologischen Probe von besagten Patienten, wobei besagte Probe ausgewählt ist aus der Gruppe bestehend aus einem operierten Tumor oder einer Biopsie und genauer gesagt einer Gewebstumorprobe, und
einen Schritt des Vergleichens des Expressionsniveaus von FSHR mit einem Kontrollreferenzwert, erhalten von einer Responder- und einer Nicht-Respondergruppe an Patienten.

2. Das Verfahren gemäß Anspruch 1, wobei der TKI ausgewählt ist aus der Gruppe bestehend aus Axitinib, Cediranib, Dasatinib, Imatinib, Nilotinib, Pazopanib, Semaxanib, Sorafenib, Sunitinib, Vandetanib, Vatalanib, Everolismus, Sirolimus und Tensirolimus.

3. Das Verfahren gemäß Anspruch 2, wobei der TKI Sunitinib ist.

4. Das Verfahren gemäß irgendeiner der Ansprüche 1 bis 3, wobei der Tumor ein fester Tumor ist.

5. Das Verfahren gemäß Anspruch 4, wobei der feste Tumor ausgewählt ist aus der Gruppe bestehend aus Nierenkrebs, wie z.B. einem Nierenzellkarzinom (RCC), Magenkrebs und Magen-Darm-Krebs, wie z.B. einem gastrointestinalen Stromatumor (GIST), Leberkrebs, wie z.B. Leberzellkarzinom (HCC), Brustkrebs, Lungenkrebs, Kolorektalkrebs, Melanom, Prostatakrebs und Bauchspeicheldrüsenkrebs, wie z.B. Bauchspeicheldrüsen-Neuroendokrintumor ist.

6. Das Verfahren gemäß Anspruch 5, wobei das Nierenzellkarzinom RCC ein metastatisches RCC ist.

7. Das Verfahren gemäß irgendeinem der Ansprüche 1 bis 6, wobei das Niveau von FSHR bestimmt wird durch Quantifizieren des Niveaus der Proteine kodiert in der Probe.

8. Das Verfahren gemäß irgendeinem der Ansprüche 1 bis 6, wobei das Niveau von FSHR bestimmt wird durch Quantifizieren des Niveaus von mRNA in der Probe.

9. Ein TKI zur Verwendung in der Behandlung eines Patienten, der unter einem Tumor leidet, umfassend den diagnostischen Schritt der Vorhersage der Reaktionsfähigkeit des Patienten auf die Behandlung mit einem TKI gemäß den Ansprüchen 1 bis 8.

10. Die Verwendung von FSHR als Surrogat-Marker zur Vorhersage der Reaktionsfähigkeit eines Patienten, der unter einem Tumor leidet, auf eine Behandlung mit einem TKI.

11. Ein Verfahren zur Vorhersage der Reaktionsfähigkeit eines Patienten, der unter einem Tumor leidet, auf eine Behandlung mit einem Tyrosinkinaseinhibitor umfassend den Schritt bestehend aus Bestimmung der Dichte von FSHR-positiven Gefäßen in der Tumorgewebsprobe, erhalten von dem Patienten.

12. Das Verfahren gemäß Anspruch 11, welches die Schritte umfasst bestehend aus i) Bestimmen der Dichte der FSHR-positiven Gefäße in der Tumorgewebsprobe, erhalten von dem Patienten und ii) Vergleichen der Dichte im Schritt i) mit einem Referenzwert, wobei der Unterschied zwischen Dichte und besagtem Referenzwert indikativ dafür ist, ob der Patient auf de Behandlung mit besagtem Tyrosinkinaseinhibitor reagieren wird.

## Revendications

1. Procédé de prédiction de la réactivité d'un patient affecté d'une tumeur à un traitement par un inhibiteur de la tyrosine kinase (TKI), comprenant une étape de mesure du niveau d'expression du FSHR dans un échantillon biologique dudit patient, ledit échantillon étant sélectionné dans le groupe constitué d'une tumeur réséquée, ou d'une biopsie, et plus spécifiquement d'un échantillon tissulaire de tumeur et d'une étape de comparaison du niveau d'expression du FSHR à des valeurs de référence témoins obtenues auprès d'un groupe de patients répondants et non répondants.

2. Procédé selon la revendication 1, le TKI étant sélectionné dans le groupe constitué de l'axitinib, du cédiranib, du dasatinib, de l'imatinib, du nilotinib, du pazopanib, du sémaxanib,du sorafénib, du sunitinib, du vandétanib, du vatalanib, de l'évérolimus, du sirolimus et du tensirolimus.

3. Procédé selon la revendication 2, le TKI étant le sunitinib.

4. Procédé selon l'une quelconque des revendication 1 à 3, la tumeur étant une tumeur solide.

5. Procédé selon la revendication 4, la tumeur solide étant sélectionnée dans le groupe constitué du cancer du rein tel que le carcinome à cellules rénales (CCR), du cancer de l'estomac et du cancer gastrointestinal tel que la tumeur stromale gastro-intestinale (TSGI), du cancer du foie tel que le carcinome hépatocellulaire (CHC), du cancer du sein, du cancer du poumon, du cancer colorectal, du mélanome, du cancer de la prostate et du cancer pancréatique tel qu'une tumeur neuroendocrine du pancréas.

6. Procédé selon la revendication 5, le cancer du rein CCR étant un CCR métastasique.

7. Procédé selon l'une quelconque des revendications 1 à 6, le niveau de FSHR étant déterminé par quantification du niveau des protéines codées dans l'échantillon.

8. Procédé selon l'une quelconque des revendications 1 à 6, dans lequel le niveau de FSHR est déterminé par la quantification du niveau d'ARNm dans l'échantillon.

9. TKI pour utilisation dans le traitement d'un patient affecté d'une tumeur, comprenant l'étape diagnostic de prédiction de la réactivité du patient au traitement par un TKI selon les revendications 1 à 8.

10. Utilisation de FSHR comme marqueurs de substitution pour la prédiction de la réactivité d'un patient affecté d'une tumeur à un traitement par un TKI.

11. Procédé de prédiction de la réactivité d'un patient affecté d'une tumeur à un traitement par un inhibiteur de la tyrosine kinase comprenant l'étape consistant à déterminer la densité des vaisseaux positifs au FSHR dans l'échantillon tumoral de tissu obtenu du patient.

12. Procédé selon la revendication 11 qui comprend les étapes consistant à i) déterminer la densité des vaisseaux positifs au FSHR dans l'échantillon tumoral de tissu obtenu du patient et ii) comparer la densité de l'étape i) à une valeur de référence dans laquelle une différence entre la densité et ladite valeur de référence est indicatrice du fait que le patient répondra au traitement par ledit inhibiteur de la tyrosine kinase.
